(19) Europäisches Patentamt
European Patent Office
Office européen des brevets

(11) Publication number: **0 331 404 A2**

(12) # EUROPEAN PATENT APPLICATION

(21) Application number: 89301942.2

(51) Int. Cl.[4]: **C12N 5/00 , C12P 17/12**

(22) Date of filing: 27.02.89

(30) Priority: 26.02.88 JP 42233/88
18.03.88 JP 63176/88
24.03.88 JP 68304/88
24.03.88 JP 68305/88
26.05.88 JP 127049/88
08.07.88 JP 169076/88

(43) Date of publication of application:
06.09.89 Bulletin 89/36

(84) Designated Contracting States:
AT BE CH DE ES FR GB GR IT LI LU NL SE

(71) Applicant: SEITAIKINOU RIYOU KAGAKUHIN SHINSEIZOUGIJUTSU KENKYU KUMIAI
13-21, Nihonbashi Kayaba-cho 1-chome
Chuo-ku Tokyo 103(JP)

(72) Inventor: Deno, Hiroshi
c/o Mitsui Petrochem. Ind., Ltd, 1-2, Waki 6-chome
Waki-cho Kuga-gun Yamaguchi-ken(JP)
Inventor: Yukimune, Yukihito
c/o Mitsui Petrochem. Ind., Ltd, 1-2, Waki 6-chome
Waki-cho Kuga-gun Yamaguchi-ken(JP)
Inventor: Itou, Aya
c/o Mitsui Petrochem. Ind., Ltd, 1-2, Waki 6-chome
Waki-cho Kuga-ken Yamaguchi-ken(JP)
Inventor: Kitani, Shigekazu
c/o Mitsui Petrochem. Ind., Ltd. 1-2, Waki 6-chome
Waki-cho Kuga-gun Yamaguchi-ken(JP)
Inventor: Motoyama, Yoshio
c/o Mitsui Petrochem. Ind.,Ltd 1-2,Waki 6-chome
Waki-cho,Kuga-gun,Yamaguchi-ken(JP)

(74) Representative: Myerscough, Philip Boyd et al
J.A.Kemp & Co. 14, South Square Gray's Inn
London, WC1R 5EU(GB)

(54) **Method for producing tropane alkaloid.**

(57) Methods for producing tropane alkaloids, such as scopolamine and/or hyoscyamine, by the tissue culture of tissues or cells of plants; tropane alkaloid-hyperproducing cells suitable for the production of tropane alkaloids, and a method for obtaining such cells; and a method for separating and purifying a tropane alkaloid from tropane alkaloid-containing cells are disclosed.

FIG.1

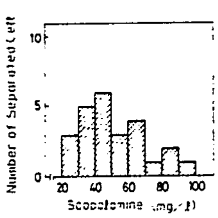


EP 0 331 404 A2

Xerox Copy Centre

# METHOD FOR PRODUCING TROPANE ALKALOID

## FIELD OF THE INVENTION

The present invention relates to a method for producing tropane alkaloids such as scopolamine and/or hyoscyamine, etc. by tissue culture of plants, a tropane alkaloid-high productive cells preferable for the production of tropane alkaloids and the method for obtaining the same, and a method for separating and purifying tropane alkaloids from a tropane alkaloid-containing substance

## BACKGROUND OF THE INVENTION

As to the tropane alkaloids, scopolamine is mainly used as an antispastics, an anodyne, and a parasympathetic system blocking agent, and hyoscyamine as a parasympathetic system blocking agent,and they are respectively used with high evaluation as medical drugs. Although these compounds are produced by extracting from natural plants, since the production of the plants are influenced by the weather, and the harvesting period is limitted, there have been problems. Therefore, many researches on the production of these compounds by the tissue culture of plants have been widely carried out in the inland and foreign countries. As to the production by use of callus, an example of the production by use of the callus of hyoscyamus by Yamada et al. is known. [Plant Cell Reports 1, 101 - 103 (1982)], but the scopolamine content was so low as 20 ppm in comparison with the content in natural plants. Also, Yamada et al. found that markedly large amounts of scopolamine and hyoscyamine were present in the adventitious roots obtained by the tissue culture of Duboisis Leich-hardtii F. Muell [ Plant Cell Reports 3, 186-188 (1984)], but the amount was yet unable to be said as sufficient. Therefore, the present inventors investigated on the various kinds of conditions in the culture of adventitious roots of Duboisia, and found that the productivity of tropane alkaloids was improved by making the ratio of ammonium ions and nitrate ions in the culture medium be 0.2 or more, and the concentration of dissolved oxygen in the culture medium be 10 or 65 ppm (Japanese Patent Application Laid-Open No. 6675/1987 and 6674/1987). But, from the industrial stand point, it is desired to increase the productivity thereof further.

Therefore, it has been an important problem to increase the productivity more, in the case when the industrial production of tropane alkaloids by the tissue culture such as described above is objected.

In view of such circumstances as described above, the present inventors have investigated the method for effectively culturing the tissues and cells of the adventitious roots of tropane alkaloid-producing plants such as Duboisia, Daturae, Hasiridokoro and Hyoscymus, etc.

Also, the adventitious root of Duboisia secondarily proliferates nicotine and derivatives thereof other than the tropane alkaloids [ Plant Cell Reports 3, 186 - 188 (1984)]. In the case when nicotine and its derivatives are present in separating and purifying tropane alkaloids from adventitious roots, there occurs such a problen that the recovery percentage is remarkably reduced. On the other hand, although the adventitious roots of Scoporia, Hyoscyamus, Beladonna, and Datura do not almost secondarily proliferate nicotine and its derivatives, but had such a problem that the content of tropane alkaloids, especially, that of scopolamine was low [ J. Plant Physiol., 124, 61 (1986); Japanese Patent Application Laid-Open No. 254195/1986].

Therefore, in case when the industrial production of tropane alkaloids, especially, that of scopolamine is objected by the tissue culture such as described above, it has been important problem to acquire the adventitious roots containing no nicotine and the derivatives thereof, and having high productivity of tropane alkaloids, especially, that of scopolamine.

In view of such circumstances as described above, the present inventors have investigated to acquire the adventitious roots of plants having high productivity of tropane alkaloids, especially of scopolamine, by deriving the adventitious roots from the plants belonging to the groups of Datura, Hyoscymus, Atropa, and Scopolia producing no nicotine and no derivatives thereof.

As to the conventional methods for separating and recovering tropane alkaloids from a tropane alkaloid-containing substance, there are reported some methods, for example, such ones by use of seeds and leaves of Hyoscyamus niger Linne, seeds of Datura tatula Linne, and D. stramonium, and leaves of Scopilia Japonica Maximowicz, and the other ones using the adventitious roots obtained by the tissue culture of plant tissues of Atropa group plants or the like, or of said tissues. For example, Szabeiska and Barbara

reported that the method is preferable in which chloroform containing 2-$FC_6H_4CO_2H$ is ued as an extracting solvent in the case of extracting alkaloid from a water layer containing alkaloids such as scopolamine, etc. [ Chemical Abstract vol. 96, (1982) ]. Also, Yu,Yongxiang [ C.A. vol. 96, (1982) ] reported that halogenised hydrocarbon, alkane, ether, and ketone are preferable as extracting solvents.

On the other hand, since various kinds of alkaloids are obtained as a mixture, when extracted by the use of these organic solvents only, Cai, Dingguo, et al. [( C.A. vol. 104, (1986)] reported a method for separating an objective alkaloid from an alkaloid mixture by the counter current partitioning chromatography using a phosphoric acid buffer solution of pH 6.2 to 6.5 as a fixed phase, and chloroform as the displacement phase.

The present inventors have examined the conventional methods in relation to the separation and purification of alkaloids and have obtained the following conclusion. That is, although the method of Szabelska, Barbara, et al. uses chloroform containing 2-$FC_6H_4CO_2H$ as a solvent for extracting alkaloids from a water layer containing alkaloids, but when extraction is carried out by using such a solvent, substances other than alkaloids such as fatty materials, polyamines, proteins and the like are accompanyingly extracted, so that the extraction selectivity for the objective alkaloids becomes low, and in the case of adding HBr in the final procedure to separate out scopolamine•3H20•HBr, the purity of the crystal becomes low, and there occurs such a defect that a high purity product is difficultly obtained by a simple process such as recrystalisation. Also, in case when a purification method in industrial scale is considered, chloroform becomes to be used in a large mass, which is not preferable from the consideration of the toxicity and the like thereof.

On the other hand, although Yu, Yongxiang, et al. uses ether, ketone, or alkane, other than chloroform as the extraction solvent of alkaloids, in case when these solvent were used, the extraction selectivity for the alkaloids is low, since substances other than alkaloids are accompanyingly extracted as described previously. Moreover, it can also be cited as a defect that the separation of the water layer and the organic solvent layer is difficult due to the generation of emulsion in extraction.

On the other hand, Cai, Dingguo, et al. proposed a method for obtaining an objective alkaloids from a mixture of alkaloids by means of a counter current partition chromatography. But, since the treating procedures become complicated, and moreover, there is a limit in the treating amount, it has problems as an industrial large amount purification method. Therefore, the present inventors have eagerly investigated a method for separating and purifying alkaloids from a tropane alkaloid-containing substance with good yield and high purity by a simple industrial process with few procedures as a novel separating recovering method different to the one described above.

## DISCLOSURE OF THE INVENTION

The object of the present invention is to provide a method for further increasing the productivity in the tissue culture of a plant producing tropane alkaloids.

Also, the object of the present invention is to provide tropane alkaloid-high productive cells and a method for obtaining them.

Furthermore, the object of the present invention is to provide a method for separating and purifying high purity tropane alkaloids simply and effectively.

At first, on the method for producing tropane alkaloids of the present invention, explanation will be given in detail in the following.

According to the present invention, there are provided the method for producing tropane alkaloids characterised by producing tropane alkaloids by effecting tissue culture of the tissues or cells of a plant producing tropane alkaloids by use of a culture medium containing oligopeptide including phenylalanine as a constitutive amino acid or a culture medium containing 1.0 $\mu$m or more of phenyl alanine derivative, and a method for producing tropane alkaloids characterised by producing tropane alkaloids by that, in the case of effecting tissue culture of the tissues or cells of a plant producing tropane alkaloids by use of a culture medium containing phenylalanine and/or its analog substance, the amount of addition at a time of the phenyl-alanine or its analog substance is made as 0.5 mM or less, and is successively added for plural times throughout the culturing period in effecting the tissue culture.

As the plants for producing tropane alkaloids used in the above-described invention can be concretely exemplified by the eggplant family plants such as the Duboisia group plants such as Duboisia myoporoides, Duboisia leichhardtii, etc., Datura group plants such as Datura tatula, Datura arborea, Datura stramonium, etc., Scopolia group plants such as Scopolia japonica, etc., Hyoscyamus group plants such as Hyoscyamus

niger, etc., and Atropa group plants such as Atropa belladonna, etc.

The oligopeptide containing phenylalanine as a constitutive amino acid according to the present invention is a peptide represented by the general formula (1)

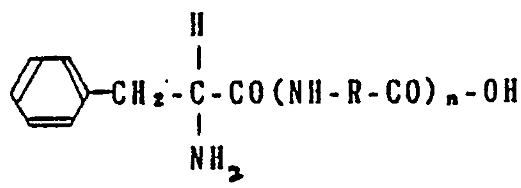

(1)

( In the formula, R denotes various kind of amino acid residual groups, n an integer 1 to 5. )

Concretely, the peptides can be exemplified as phenylalanyl-methionine, diphenyl-alanine, phenylalanylproline, phenylalanyl-thyrosin, phenylalanyl-alanine, triphenyl alanine, etc.

In the present invention, although the ratio of the use of the above-described oligopeptide contained in the culture medium differs in dependence to the kind of oligopeptide used, it is in general 0.5 µM to 500 µM, or preferably, in the range of 1 µM to 100 µM.

As the phenyl alanine analog substance according to the present invention, can be used the derivative of amino acid which is represented by the general formula (I).

$$(R^1)_n \; C_6H_{5-n} \; -CH_2-\overset{\displaystyle NHR^2}{\overset{|}{CH}}-COR^3 \qquad (I)$$

(In the formula, $R^1$ denotes a low grade alkyl group or a halogen atom, $R^2$ a hydrogen atom or $R-\overset{O}{\overset{\|}{C}}-$( R is H or a low grade alkyl group ), $R^3$ OH, $NH_2$, or OM ( M is a metal ion ), and n an integer 1 to 3.)

Concretely, the substance can be exemplified as alkylphenyl alanine such as 4-methyl-phenyl alanine or the like, halogenised phenylalanine such as 4-chloro-phenylalanine, 4-fluorophenylalanine, etc., and amino derivatives such as 4-fluorophenyl alanine amide etc. In the present invention, among these compounds, the carboxyl group in said compound may be such a one as to form a salt with the same metal ion as the inorganic constituent described in the following, and in the present invention, this salt can be used as the constituent of the culture medium.

Also, as the phenyl alanine analog substance, may be used β-2-thienyl alanine, and the like.

In case when the content of the phenyl alanine analog substance in the culture medium is 1.0 µM or less, the formation amount of the tropanealkaloids is not improved so much, even if tissue culture is carried out by using such a culture medium, so that in the present invention, tissue culture is effected by use of a culture medium in which the content of said constituent is made as 1.0 µM or more. In said range, the range of 2 µM to 30 µM is especially preferable. The upper limit of the phenyl alanine analog substance concentration is usually 1 mM.

Also, in the present invention, in the case of effecting tissue culture of the tissues or cells of the above-described plants by use of a culture medium containing phenyl alanine and/or the analog substance thereof, the amount of phenyl alanine and/or the analog substance thereof added at a time is made as 0.5 mM or less, and throughout the cultural period, the addition is effected successively for plural times, and thus, the productivity of the tropane alkaloids can be increased.

In this case, the total sum of the all added amounts of the phenyl alanine and/or the analog substance thereof added to the culture medium during the period of the tissue culture is, when represented, for example, per 10 mg of the dried weight of the tissues of said plants is such as the adventitious roots or the like provided for the culture, usually in the range of 0.2 to 2.0 mM.

In the present invention, the culture is effected in such a manner as that this total addition amount is divided into plural times, and moreover, such as to make the addition amount per one time be 0.5 mM or less. In the present invention, the word "plural times" means two or more times.

In the present invention, since the growth amount of cells becomes lowered when the addition amount

at one time becomes 0.5 mM or more, and in severe cases, necrosis of cells occurs to lower the formation amount of scopolamine and hyoscyamine, so that the addition amount per one time is made as 0.5 mM or less. Although there is no lower limit in the addition amount for once, but when the addition amount is too little, the productivity of scopolamine or the like becomes lowered and is not economical, so that the addition is effected usually in the range of 0.001 mM to 0.5 mM, or preferably, in the range of 0.01 mM to 0.3 mM.

In the culture medium used in the present invention, as to the culture medium constituents other than the above-described oligopeptide, phenyl alanine, or the above-described phenyl alanine analog substances, can be cited inorganic constiuents such as inorganic salts containing the element such as nitrogen, phosphorous, potassium, sodium, calcium, magnesium, sulfur, iron, manganese, zinc, boron, molybdenum, chlorine, iodine, cobalt, etc. Concretely, can be exemplified by such compounds as potassium nitrate, sodium nitrate, ammonium nitrate, ammonium chloride, potassium chloride, calcium chloride, phosphoric acid mono-hydrogen potassium, phosphoric acid di-hydrogen potassium, magnesium sulfate, magnesium chloride, sodium sulfate, ferrous sulfate, ferric sulfate, manganese sulfate, copper sulfate, sodium molybdate, molybdenum trioxide, potassium iodide, zinc sulfate, boric acid, cobalt chloride, etc.

As the carbon source of said culture medium can be cited for example, carbohydrates such as cane sugar or the like and the derivatives thereof, organic acids such as aliphatic acids, and primary alcohols such as ethanol or the like.

As the plant hormones, can be cited as examples auxins such as naphthalene acetic acid (NAA), indole acetic acid (IAA), p-chlorophenoxy acetic acid, 2, 4-dichlorophenoxy acetic acid (2, 4-D), indole butyric acid (IBA), and the derivatives thereof, etc., and cytokinins such as benzyl adenin (BA), kinetin, zeatin, or the like. In the present invention, although it is usually desirable that cytokinins are not added to the culture medium, but in case when they are added in correspondence to needs, it is preferrable to use cytokinins at a low concentration such as in general at a concentration of $10^{-7}$M (0.02 mg/l) or less.

As the vitamins for said culture medium, can be exemplified such ones as biotin, thiamine ( vitamin B1 ), pyridoxine ( vitamin B6 ), pyridoxal, pyridoxamine, calcium panthothenate, ascorbic acid ( vitamin C ), inositol, nicotinic acid, nicotinic acidamide, and liboflavin ( vitamin B2 ).

As the amino acids of said culture medium can be exemplified, for example, glycine, alanine, glutamic acid, and ligin.

It is desirable that the above-described culture medium of the present invention is used in general by being contained with said inorganic constituent for about 0.1 $\mu$M to about 100 mM, said carbon source for about 1 g/l to about 100 g/l, said plant hormones for about 0.01 $\mu$M to about 100 $\mu$M, said vitamins for about 0.1 mg/l to about 150 mg/l, and said amino acids for 0 to about 100 mg.

As said culture medium used in the tissue culture of the Duboisia group plants of the present invention, can be exemplified concretely the culture medium heretofore known and used in the tissue culture of plants such as, for example, Murashige & Skoog culture medium ('62), Linsmaier & Skoog culture medium (RM-1965), White culture medium ('63), Gamborg B-5 culture medium, Mitsui M-9 culture medium, Nitch•Nitch culture medium, etc. To such culture medium as described above, oligopeptide, phenyl alanine or phenyl alanine analog substances according to the present invention and the above-described carbon source and plant hormones are added, and further, as occasion demands, said vitamins and amino acids are added to prepare the culture medium. In the present invention, among the culture mediums described above, especially preferable is the one prepared by use of the Nitch•Nitch, Rinsmaier & Skoog, or Murashige Skoog culture medium. By the way, as to the composition of the above-described conventional known culture medium, description is found in the book written by Takeuchi, Nakajima, and Furuya: "New Plant Tissue Culture," p. 386 to p. 391, Asakura Shoten, (1979).

The above-described culture medium usable in the present invention is a liquid culture medium or a solid culture medium containing agar or gelatin for the concentration of usually 0.5 to 1%, but in the present invention, the use of a liquid culture medium is preferable.

As tissues or cells of the above-described plants used in the tissue culture of the present invention, other than the tissue fractions or cells such as roots, leaves, stems, seeds, flower buds of said plants, cultured cells or cultured tissues of said plants obtained by the tissue culture according to the present invention or by the other conventional tissue culture method can be exemplified. In the present invention, it is especially preferable to effect tissue culture by the use of the adventitious roots obtained by previously effecting the tissue culture of plant tissues by using the culture medium according to the present invention. In this case, the adventitious roots of the raw material can be increasingly cultured by the use of the culture medium of the present invention, and adventitious roots containing large amount of tropane alkaloids can be obtained.

Also, in the presènt invention, it is also preferable to use such a method that callus is derived from the

above-described tissue fractions or cells, and the cultured cells or cultured tissues obtained by passage culture of said callus is proliferately cultured by the use of said culture medium of the present invention to obtain cultured material containing large amount of tropane alkaloids, especially, adventitious roots.

In case when adventitious roots are used in the present invention, the tissue fraction is made be infected with the hair root germs ( for example, Agrobacterium rhizogenes ) and can use the hair roots revealed thereby. ( For example, it is also possible to use the method provided by Japanese Patent Application Laid-Open No. 248429/1987 according to the present applicant. )

As the tropane alkaloids obtainable by the method of the present invention, can be exemplified concretely, scopol amine, hyoscy amine, and acetyl compounds of these compounds, but, among such ones, scopol amine and hyoscy amine are preferable.

In the present invention, as the method for separating alkaloid from cultured cells containing tropane alkaloids, can be adopted the usual method described, for example, in the pharmacopoeia or the like such as the one used in separating and purifying these compounds from the tropane alkaloid-containing plants.

When the method for producing tropane alkaloids by the tissue culture of the present invention described above in detail is adopted, it is possible to produce tropane alkaloids, especialy among them, scopol amine and/or hyoscy amine in large amount and effectively in comparison with the case of conventional methods.

Next, explanation will be given on the tropane alkaloids high productivity cells derived from the tropane alkaloid-producing plants of the present invention and show resistivity to phenyl alanine or phenyl alanine analog, and the method for obtaining the same.

In general, it is known that, in cells showing resistivity to amino acid analogs in the tissue culture of plants, there are present such cells having high content of amino acid.

For example, J.M. Widholm et al. found that, in the cell culture of carrots, the stock which is tolerant to ethionine, as the analog of methionine, includes 10 times amount of methionine contained in normal cells. [ Can. J. Botany, 54, p. 1523 (1976)]. On the fact that the excess production of amino acid occurs in such amino acid resistive stocks, explanation is given in the book edited by Yasuyuki Yamada " Variation and Selection of Plant Culture Cells " ( Kodansya Scientific (Biotechnology Series); p. 37 to p. 38), in such a way as that the amino acid concentration in cells is increased in order to cope with the inhibitting action of the amino acid metabolism by amino acid analog.

Therefore, the present inventors noticed such a phenomenon as described above, and have investigated on the method of effectively selecting the tropane alkaloid high productive cells from the tissues and cells of the adventitious roots and the like of tropane alkaloid-producing plants, and have found the following facts.

That is, by treating the tissue or cells with the phenyl alanine or the analog substance thereof which are the precursors of tropane alkaloids, phenylalanine tolerant stocks or phenyl alanine analog tolerant stocks were obtained, and the present inventors have found the presence of the stocks producing tropane alkaloids at a high content among said tolerant stocks in a high ratio, and have attained to the completion of the present invention.

According to the present invention, there is provided a method for obtaining tropane alkaloid high productive cells characterised by forming stocks showing tolerance to phehyl alanine or phenyl alanine analog by use of tropane alkaloid high productive cells derived from tropane alkaloid-producing plant and showing tolerance to phenyl alanine or phenyl alanine analog, and the tissues or cells of tropane alkaloid-producing plant in a culture medium containing phenyl alanine or phenyl alanine analog, and by selecting out the high productive cells from the tolerant cells obtained.

As the tropane alkaloid-producing plants used in the present invention can be concretely exemplified the plants described in the above-described explanation on the method for producing tropane alkaloids.

In order to obtain the tropane alkaloid high productive cells, it is necessary to form the stocks which show tolerance to phenyl alanine or the analog thereof by effecting, at first, the culture of the cultured tissue of the above-described plant in the culture medium containing phenyl alanine or the analog thereof for more than a predetermined period. In the following, description will be given thereon in detail.

The culture medium for making phenyl alanine or its analog tolerant stocks is the culture medium containing phenyl alanine or its analog as a necessary componenent.

As the phenyl alanine analog used in the invention related to the tropane alkaloid high productive cells and the method for obtaining thereof can be cited the compounds shown by the above-described general formula [1]. Also, $\beta$-2-thienyl alanine and the like can be used.

As the culture medium constituents other than the above-described phenyl alanine or the analog thereof, inorganic constituent and carbon source are the necessary constituents, and plant hormones and vitamins are added thereto, and further, amino acids are added in needs.

In the present invention, as the use ratio of the above-described phenyl alanine or the analog thereof, the range of $10^{-5}$M to $10^{-1}$M is preferable, and in said range, the range of $10^{-4}$M to $10^{-2}$M is especially preferable. When the concentration of phenyl alanine or the analog thereof is higher than the one in this range, all cells die out, and phenyl alanine or the analog-tolerant stock thereof becomes unable to be obtained. Also, in the concentration lower than that of this concentration, cells which are not tolerable to phenyl alanine or the analog thereof also grow up, and the phenyl alanine analog-tolerant stock becomes unable to be obtained.

In case when phenyl alanine or its analog tolerant-stock is to be obtained, it is desirable to effect passage culture, in which the culture period for one generation is in general one month, for more than two generation in a culture medium containing phenyl alanine or the analog thereof. When the period is shorter than the one described above, cells become unable to acquire the tolerance for phenyl alanine or the analog thereof.

In the culture medium used in the present invention, for the culture medium components other than the above-described phenyl alanine or the analog thereof, can be used the similar ones as those explained above in the method for producing tropane alkaloids, that is, inorganic constituents, carbon sources, plant hormones, vitamins, and amino acids.

The above-described culture medium of the present invention is in general desirable to be used by being contained with said inorganic constituent for about 0.1 $\mu$M to about 100 mM, said carbon source for about 1 g/l to about 100 g/l, said plant hormones for about 0.01 $\mu$M to about 100 $\mu$M, said vitamins for about 0.1 mg/l to about 150 mg/l, and said amino acids for 0 to about 100 mg/l.

As the above-described culture medium of the present invention, can be concretely exemplified such ones as the culture medium used in the tissue culture of heretofore known plants, for example, the culture mediums prepared by adding above-described carbon sources and plant hormones, and further adding above-described vitamins, amino acids, in correspondence to needs to such culture mediums as the Murashige & Skoog '62 culture medium, Linsmaier & Skoog RM-1965 culture medium, White '63 culture medium, Gamborg B-5 culture medium, Mitsui M-9 culture medium, Nitch•Nitch culture medium, etc.

In the present invention, among these ones, the culture mediums prepared by the use of the Nitch•Nitch culture medium, Linsmaier & Skoog culture medium, or Murashige & Skoog culture medium is especially preferred. By the way, as to the conventional known culture mediums, description are found in the book written by Takeuchi, Nakajima, and Furuya " New Plant Tissue Culture " p. 386 to p. 391, Asakura Shoten, (1979).

Although the above-described culture medium capable of being used in the present invention is a liquid culture medium or the solid culture medium made be contained in general with agar, gelatin, or the like for 0.5 to 1%, but in case when phenyl-alanine or its analog tolerant stocks are made, the use of a solid culture medium is preferable. Also, in case when culture is effected in order to produce tropane alkaloids by use of said tolerant stocks, and in the culture for maintaining said tolerant stocks, the use of a liquid culture medium containing no phenyl alanine nor its analog is preferable.

In the present invention, as the tissues or cells of the above-described plants used in the case of producing the phenyl alanine or its analog tolerable cells, can be concretely exemplified, other than the tissue fractions or cells such as the roots, leaves, stems, seeds, flower buds, etc. of said plants, the cultured cells or cultured tissue of the above-described plants obtained by the conventional method for tissue culture. In the present invention, among these ones, it is especially preferable to use the adventitious roots obtained by previously effecting tissue culture of the plant tissue to effect tissue culture by the use of the culture medium according to the present invention. Also, the cultured cells or cultured tissues obtained by the passage culture of the callus derived from the above-described tissue fractions or cells can be used.

In the present invention, in case of the use of adventitious roots, it is possible to infect the tissue fractions of a plant to hair root desease bacteria ( for example, Agrobacterium rhizogenes ), and the hair roots appeared thereby are used. ( For example, the method proposed by the present applicant in Japanese Patent Application No. 89975/1986 can be used.)

In such a manner as described above, cells showing tolerance to phenyl alanine or to its analog were produced and the production amount of tropane alkaloid of the tolerant cells obtained was measured, and by selecting the cells having high production amount of tropane alkaloids therefrom, tropane alkaloid high productive cells can be obtained.

By the tissue culture of the tropane alkaloid high productive cells according to the present invention in the culture medium, cultured product containing large amount of tropane alkaloid can be obtained. As the culture medium to be used, the Nitch•Nitch culture medium containing IBA $10^{-5}$M or Linsmaier & Skoog culture medium are preferable.

As the tropane alkaloids obtainable by the method of the present invention, can be exemplified

concretely such compounds as scopolamine, hyoscyamine, and acetyl compounds of these compounds, but among these ones scopolamine and hyoscyamine are preferable.

In the present invention, as the method for separating alkaloid from cultured cells containing tropane alkaloids, a usual method can be adopted, which is described in the pharmacopeia and the like and is used in separating and purifying these com pounds from the plants containing tropane alkaloids.

According to the present invention, a novel tropane alkaloid high productive cells can be provided, and tropane alkaloid high productive cells can be effectively obtained. When the tropane alkaloid high productive cells of the present invention are used, in the tropane alkaloids, espedially, the productivity of scopolamine and hyoscyamine can be enhanced.

Next, in the production method of tropane alkaloids by culturing the adventitious roots of tropane alkaloid-producing plants of the present invention, the production method of tropane alkaloids will be explained, in which Datura arborea L. is used as the above-described plant.

It is known that the adventitious roots of the plant belonging to Datura group produce tropane alkaloids, but the productivity thereof, especially the productivity of scopolamine has been usually very low, [Japanese Patent Application Laid-Open No. 254195/1986, Japanese Patent Application Laid-Open No. 205792/1987 ]. The present inventors have found that the adventitious roots derived from Datura arborea belonging to the Datura group produce especially large amount of scopolamine, and do not secondarily produce nicotine and the derivatives thereof, and have attained the completion of the present invention. By the way, as to this adventitious root culture, quite no report is known heretofore on the fact that these adventitious roots produce tropane alkaloids, especially, scopolamine to an extremely large amount in comparison with the cases of the adventitious roots of the other plants of the Datura group, Hyoscyamus group, Atropa group, and Scopolia group. From such findings as described above, the present invention has been completed.

According to the present invention, a method for producing tropane alkaloids is provided, which is characterised by producing tropane alkaloid, especially, scopolamine by the tissue culture of adventitious roots derived from Datura arborea.

In the present invention, tissue culture is carried out by use of the adventitious roots of Datura arborea.

The culture medium used in the present invention contains inorganic constituents and carbon sources as necessary constituents, and is added with plant hormones and vitamins at need, and further amino acids.

As inorganic constituents, can be cited inorganic salts containing such elements as nitrogen, phosphorous, potassium, sodium, calcium, magnesium, sulfur, iron, manganese, zinc, boron, molybdenum, chlorine, iodine, cobalt, etc., and concretely, can be exemplified as such compounds as potassium nitrate, sodium nitrate, ammonium nitrate, ammonium chloride, potassium chloride, calcium chloride, phosphoric acid monohydrogen potassium, phosphoric acid dihydrogen potassium, magnesium sulfate, magnesium chloride, sodium sulfate, ferric sulfate, ferrous sulfate, manganese sulfate, copper sulfate, sodium molybdenate, molybdenum trioxide, potassium iodide, zinc sulfate, boric acid, cobalt chloride, etc.

As carbon sources of said culture medium can be exemplified as the carbohydrate such as cane sugar and the like and the derivatives thereof, organic acids such as aliphatic acids and the like, and primary alcohols such as ethanol, etc.

As plant hormones of said culture medium, can be exemplified, for example, auxines such as naphthalene acetic acid (NAA), indole acetic acid (IAA), p-chlorophenoxy acetic acid, 2, 4-dichlorophenoxy acetic acid (2, 4-D), indolebutyric acid (IBA), and the derivatives of these ones, and cytokinines such as benzyladenine (BA), kinetin, zeatin, etc. In the present invention, although it is desirable that cytokinines are not usually added to the culture medium, but when it is added in need, it is preferable that cytokinenes are used at a low concentration of below $10^{-7}$ M (0.02 mg/l) in general.

As the vitamins of said culture medium, can be exemplified such ones as biotin, thiamine (vitamin $B_1$), pyridoxin (vitamin $B_6$), pyridoxal, pyridoxamine, calcium pantothenate, ascorbic acid (vitamin C), inositol, nicotinic acid, nicotinic acid amide, and riboflavin (vitamin B2), etc.

As the amino acids of said culture medium, can be exemplified, for example, glycine, alanine, glutamic acid, phenylalanine, lysine, etc.

The above-described culture medium of the present invention is desirably be used by mixing about 0.1 $\mu$M to about 100 mM of said inorganic constituents, about 1 g/l to about 100 g/l of said carbon sources, about 0.01 $\mu$M to about 100 $\mu$M of said plant hormones, about 0.1 mg/l to about 150 mg/l of said vitamines, and o to about 100 mg/l of said amino acids.

As said culture medium used in the tissue culture of the present invention, can be concretely exemplified the culture medium prepared by adding the above-described carbon sources and plant hormones, and further,by adding above-described vitamins, amino acids, and the like in need to a culture medium used in the tissue culture of hitherto known plants, such as, for example, Murashige & Skoog '62

culture medium, Linsmaier & Skoog RM-1965 culture medium, White '63 culture medium, Gumborg B-5 culture medium, Mitsui M-9 culture medium, Nitch•Nitch culture medium, etc. But, in the present invention, among these ones, the culture mediums prepared by using the Nitch•Nitch, Linsmaier & Skoog, or Murashige & Skoog culture medium is preferable. By the way, as to the composition of the above-described conventionally known culture medium, description is found , for example, in the book written by Takeuchi, Nakajima, and Furuya " New Plant Tissue Culture ", p. 386 to p. 391, Asakura Shoten (1979).

The above-described culture medium usable in the present invention is a liquid culture medium or a solid culture medium containing agar or gelatin for 0.5 to 1% in general, but in the present invention the use of liquid culture medium is preferable.

The adventitious roots used in the tissue culture of the present invention can be derived by culturing the root, stem, leaves, seeds, flower buds, of said plant and the callus derived from these tissues in a culture medium containing auxines such as indole acetic acid or the like, and adventitious roots containing large amount of tropane alkaloids can be obtained by proliferous culture by the use of the culture medium as described above.

In the present invention, in case when adventitious roots are used, the tissue fractions of a plant is infected, for example, to a hair root desease bacteria (for example, Agrobacterium rhizogenes), and the hair roots appeared thereby can be used also. (For example, the nethod provided by the present applicant in Japanese Patent Application Laid-Open No. 248249/1987 can also be used.)

As the tropane alkaloid obtainable by the method of the present invention, can be concretely exemplified scopolamine, hyoscyamine, and the acetyl compound of these compounds, but among these ones, scopolamine and hyoscyamine are preferable.

In the present invention, as the method for separating alkaloid from adventitious roots containing tropane alkaloids, a usual method can be adopted, which is described in the pharmacopeia and the like and has been used in the case of separating and purifying these compounds from the plant containing tropane alkaloids.

When the production method of tropane alkaloids by the tissue culture of the present invention is adopted, tropane alkaloids, especially scopolamine and/or hyoscyamine can be produced in large amount and effectively in comparison with conventional methods without secondarily producing nicotine and the derivatives thereof.

In the following, on the method for separating and purifying tropane alkaloids of the present invention, explanation will be given.

According to the method of the present invention, tissues or cells obtained by the tissue culture of tropane alkaloid-containing plant is heat treated as they are, to let the tropane alkaloid contained in the tissues or cells be released in the culture medium, and after regulating the pH of said culture medium to be in the range of 6.5 to 8.5 with a base, it is treated with aliphatic group carboxylic acid ester to obtain an extracted liquid. When the aliphatic group carboxylic acid ester as the extracting liquid is removed from this extracted liquid, syrup-like raw scopolamine can be obtained. Successively, the raw scopolamine is dissolved, for example, in ethanol, and hydrohalogenic acid is added thereto, and the solution is left stand still at 5° C for one night, and hydrohalogenic of the objected scopolamine can be deposited and separated.

The tropane alkaloid separated and purified by the method of the present invention is a compound represented concretely by the formula (1).

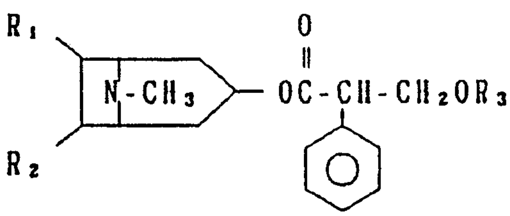

[I]

and is a tropane alkaloid such as, for example, scopolamine ( in the formula [I], $R_1$, $R_2$ = -O-, $R_3$ = H ), atropine ( in the formula [I], $R_1$, $R_2$, $R_3$ = H ), acetyl scopolamine ( in the formula [I], $R_1$, $R_2$, = -O- , $R_3$ = $CH_3CO$ ), acetyl atropine ( in the formula [I], $R_1$, $R_2$ = H, $R_3$ = $CH_3CO$ ), etc. In the present invention, among these ones, scopolamine is preferable.

In the method for separation and purification of tropane alkaloid of the present invention, the tissues and cells of the tropane alkaloid-containing plant used as raw materials are concretely the tissue fractions of egg plant family plants such as Hyoscyamus, western style Korean morning glory, Rohtocon, etc., or the adventitious roots or cultured cells obtained by the tissue culture of said plant tissue fractions. Adventitious

roots or cultured cells are usually present as a mixture with the culture medium, so that, for example, when a liquid culture medium is used as a culture medium, it is heated ( for example, at 40 to 80 °C for 2 to 12 hr ), as it is, without separating the adventitious roots or cells from the culture medium to release almost all of the tropane alkaloid to the culture medium. Adventitious roots or cells are filtered and removed, and, in order to selectively extract the objective scopol-amine with an aliphatic group carboxylic acid ester from the culture medium, the pH of the culture medium is regulated with a base to 6.5 to 8.5. As the base used for regulating the pH, ammoniacal water, $NaHCO_3$ or NaOH, etc. is preferable. Next, as the aliphatic group carboxylic acid ester used in the case of extracting scopolamine from the culture medium with an aliphatic group carboxylic acid ester can be concretely cited such ones as ethyl formate, ethyl acetate, isobutyl acetate, methyl propionate, ethyl butylate, etc. Among these ones, isobutyl acetate is especially preferable. These aliphatic group carboxylic acid esters may be used solely or as a mixed constituent.

As an industrial extracting procedure to be adopted in the present invention, can be used the tower-type counter current extraction method usibg multi-perforated plates or fillers, or a centrifugal extrac tion equipment, but the tower type counter current extraction method is especially preferable. Also, the use amount of the aliphatic group carboxylic acid esters used for extraction is good when it is 0.2 to 1.5 times by weight of the culture medium containing tropane alkaloid, and the amount of 0.3 to 0.6 times by weight is especially preferable.

As the extracting liquid (isobutyl acetate) contains a small amount of water, so that, after adding a dehydrating agent such as anhydrous sodium sulfate or the like, and being left to stand still to be removed of water, isobutyl acetate is distilled off to obtain syrup-like raw scopolamine trihydrate. This is dissolved in a polar organic solvent and is added with hydrogen halogenide and is left stand still for one night to obtain needle-like crystalls of the objective scopolamine•$3H_2O$•hydrogen halogenide.

As the polar organic solvent for dissolving the trihydrate salt of syrup-like raw scopolamine, is concretely used methanol, ethanol, isopropanol, acetone, dioxane, etc. Among these ones, ethanol is especially preferable. The amount of ethanol is good when it is 5 to 50 times by weight of the syrup-like raw scopolamine•$3H_2O$, and especially preferable when it is 5 to 10 times by weight.

As the hydrogen halogenide usable in the present invention can be exemplified hydrogen bromide, and hydrogen chloride. Among these ones, hydrogen bromide is preferable.

As the hydrogen bromide source in the case when scopolamine is made as a hydrogen bromide acid salt, the commercial 47% hydrogen bromide acid may be used as it is, or a diluted hydrogen bromide acid can be used. Also, hydrogen bromide gas may also be used. The use amount of hydrogen bromide is good at 1.1 to 10 times mol for that of scopolamine•$3H_2O$, and especially preferable at 2 to 5 times mol.

When the scopolamine•$3H_2O$•HBr obtained in the manner as described above is recrystallized in need with ethanol or the like, high purity scopolamine•$3H_2O$•HBr can be further obtained.

According to the method of the present invention, by means of a simple method having less number of procedures in comparison with conventional methods, scopolamine in tropane alkaloids can be selectively separated and purified from a tropanealkaloid-containing substance at high purity, and moreover, with good recovery percentage.

[ Embodiments ]

In the following, the method for producing tropane alkaloids by use of a specified culture medium will be explained.

Examples 1, 2 and 3

Leave of Duboisia myporoides R. Br cultivated in a herb garden of our company was cleaned and immersed in a 10% antiformin liquid for 10 minutes, successively, after being cleaned with sterilised water 3 times, the leaves were cut to about 1 cm size, and then, were placed on the Linsmaier & Skoog agar culture medium added with naphthalene acetic acid and benzyl adenine respectively to become $10^{-5}$ M and $10^{-6}$ M, and were cultivated at 25 °C for 30 days. The adventitious roots generated at the same time with the callus formation were cut out, and were transfered to a liquid Nitch•Nitch culture medium added with indole butyric acid to become $10^{-5}$ M, and passage culture was carried out for two years.

The adventitious roots (10 mg dried weight) thus obtained were transplanted into an Erlenmeyer flask of 100 ml volume containing 20 ml of Nitch•Nitch liquid culture medium added with indole butyric acid to become $10^{-5}$M, and at the time of the start of the culture, phenyl alanyl methionine was added to the

previously described liquid culturfe medium such that the phenylalanyl methionine concentration becomes respectively as 10 µM, 100 µM, and 500 µM, and further, shake cultivated for 3 weeks. After drying the adventitious roots obtained, they were extracted with 50 ml of basic chloroform-methanol solution. Then, 40 ml of 1 N sulfuric acid was added thereto to transfer the alkaloid layer to the sulfuric acid layer. Further, 2 ml of ammoniacal water and 40 ml of chloroform were added to transfer the alkaloid to the chloroform layer, and this mixture was concentrated under reduced pressure, and the alkaloid amount was analyzed on the gas chromatograph. The production amount of alkaloid in this case is shown in Table 1. By the way, the analysis on the gas chromatograph was carried out under the following conditions:

Column: Silicone OV-17 ( 1% ) on

Chromosorb W ( Mesh 80 to 100 ) 3 mm x 1 m Glass column

Carrier gas: $N_2$ ; Column temperature: 200° C

Comparative Example 1

Except that a culture medium not added with phenylalanyl methionine was used in Example 1, an experiment was carried out in the same manner as in said Example, and the results are shown in Table 1.

Examples 4, 5, and 6

In Erlenmeyer flasks of 100 ml volume containing 20 ml of Nitch•Nitch liquid culture medium added with diphenyl alanine in such amount as its concenration becomes respectively 1 µM, 10 µM, and 100 µM, and added with indole-butyric acid in an amount to become $10^{-5}$M, 10 mg of the adventitious roots obtained in the same manner as in Example 1 were transplanted. After cultivating for 3 weeks, the same treatment as in Example 1 was carried out, and the alkaloid amount was analyzed. The alkaloid production amounts in this case are shown in Table 2.

Comparative Example 2

Except that diphenyl alanine was not added in Example 4, experiment was carried out in the same manner as in said Example, and the results are shown in Table 2.

Examples 7, 8, 9, and 10

In Erlenmeyer flasks of 100 ml volume containing 20 ml of Nitch•Nitch liquid culture medium added with triphenyl alanine in such an amount that its concentration becomes respecitively 0.5 µM, 1.0 µM, 5.0 µM, and 10 µM, and added with indole butyric acid in an amount to become $10^{-5}$M, 10 mg of the adventitious roots obtained in the same manner as in Example 1 were transplanted. After cultivating for 3 weeks, the same treatment as in Example 1 was carried out, and the amounts of alkaloid was analyzed. The alkaloid amounts in this case are shown in Table 3.

Comparative Example 3

Except that triphenyl alanine was not added in Example 7, experiment was carried out in the same manner as in said Example, and the results are shown in Table 3.

Examples 11 to 14

Leaves of Duboisia myoporoides R. Br cultivated in the herb garden of our company were cleaned and immersed in a 10% antihormine liquid for 10 minutes, and successively, after cleaning for 3 times with sterilized water, they were cut to about 1 cm long pieces, and the pieces were put on a Linsmaier & Skoog culture medium added with naphthalene acetic acid and benzyl adenine to become respectively to the concentration of $10^{-5}$M and $10^{-6}$M to be cultivat ed at 25° C for 30 days. The adventitious roots generated

at the same time with the formation of callus were cut out and transplanted to the Nitch•Nitch liquid culture medium added with indole butyric acid such as to attain the concentration of $10^{-5}$M, and passage culture was carried out for 2 years. The adventitious roots thus obtained (dried weight of 10 mg) were transplanted in 100 ml volume Erlenmeyer flasks containing 20 ml of Nitch•Nitch liquid culture medium added with indole butyric acid to attain the concentration of $10^{-5}$M, and at the start of culture, 4-fluorophenyl alanine was added to the previously described liquid culture medium such as the 4-fluorophenyl alanine concentration becomes respectively to reach 1 µM, 3 µM, 10 µM, and 30 µM, and further, shaking culture was carried out for 3 weeks. After drying the adventitious roots obtained, they were extracted with 50 ml of basic chloroform-methanol mixture. To this mixture, 40 ml of 1 N sulfuric acid was added to transfer the alkaloid layer to the sulfuric acid layer. Further, 2 ml of ammoniacal water and 40 ml of chloroform were added to transfer the alkaloid into the chloroform layer to be concentrated under reduced pressure. The alkaloid amount was analyzed on gas chromatograph. The production amount of alkaloid in this case are shown in Table 4. By the way, the gas chlomatograph analysis was carried out under the following conditions.
Column: Silicone OV-17 ( 1% ) on Chromosorb W ( Mesh 80 to 100 ) 3 mm ø × 1 m Glass column
Carrier gas: $N_2$
Column temperature: 200 °C

Comparative Example 4

Except that the culture medium not added with 4-fluorophenyl alanine was used in Example 11, the experiment was carried out in the same manner as in said Example, and the results are shown in Table 4.

Comparative Example 5

Except that the 4-fluoro-phenyl alanine concentration was changed to 0.05 µM in Example 11, the experiment was carried out in the same manner as in said Example, and the results are shown in Table 4.

Examples 15 to 17

After treating the leaves of Duboisia myoporoides R. Br with 10% antihormine, they were placed on a Linsmaier & Skoog (LS) agar culture medium containing $10^{-5}$M of benzyl adenine. The stem of the Duboisia sapling, which has been obtained by effecting passage culture of the sapling generated after one month in the same culture medium for 40 days and is not yet lignified, was cut to the length of about 1 to 2 cm, and was subjected to shaking culture for 24 hours.

After immersing the sample in a suspension ($10^7$ pieces/ml) of Agrobacterium rhizogenes HRI-1, it was put on the LS agar culture medium containing no plant hormone. After 3 weeks, from the cut part of the step of the sapling, adventitious roots were generated. The roots were treated in the LS liquid culture medium containing 0.1 mg/ml of ampicyline for two days and the self-proliferating Duboisia-infected adventitious roots containing no bacteria were obtained. This Duboisia infected adventitious roots were subjected to passage culture for one year in the LS liquid culture medium. In 100 ml volume Erlenmeyer flasks containing 20 ml of the LS liquid culture medium added with 4-fluorophenyl alanine such as the concentration becomes respectively 3 µM, 10 µM, and 30 µM, 10 mg (dried weight) of the adventitious roots were transplanted and shake cultured for 3 weeks. The adventitious roots obtained were dried and extracted by the same method as in Example 1, and the alkaloid amount was analyzed. The production amount of alkaloid in this case is shown in Table 5.

Comparatine Example 6

Except that 4-fluorophenyl alanine was not added in Sxample 15, the experiment was carried out in the same manner as in said Example, and the results are shown in Table 5.

Comparative Example 7

Except that the concentration of 4-fluorophenyl alanine was made as 1 µM in Example 15, the experiment was carried out in the same manner as in said Example, and the results are shown in Table 5.

Examples 18 to 20

Into 100 ml volume Erlenmeyer flasks containing 20 ml of Nitch•Nitch liquid culture medium added with 4-chlorophenyl alanine such as the concentration becomes respectively as 3 µM, 10 µM and 30 µM, and with indole butyric acid such as its amount becomes $10^{-5}$M, 10 mg of adventitious roots obtained in the same manner as in said Example were transplanted. After culturing for 3 weeks, treatment was effected in the same manner as in said Example, and the alkaloid amount was analyzed. The alkaloid amounts in this case are shown in Table 6.

Comparative Example 8

Except that 4-chlorophenyl aniline was not added in Example 18, the experiment was carried out in the same manner as in said Example, and the results are shown in Table 6.

Comparative Example 9

Except that the concentration of 4-chlorophenyl alanine was changed as 0.5 µM, the experiment was carried out in the same manner as in said Example, and the results are shown in Table 6.

Example 21

The leaves of Duboisia myporoides B. Br cultivated in the herb garden of our company were cleaned and immersed in 10% antiformin liquid for 10 minutes, and after successively cleaned with sterilized water for 3 times, they were cut to the length of about 1 cm, and put on a Linsmaier & Skoog agar culture medium added with naphthalene acetic acid and benzyl adenine such that the amount becomes, respectively, $10^{-5}$M and $10^{-6}$M, and were cultured at 25°C for 30 days. The adventitious roots generated at the same time with the formation of callus were cut out, and were transplanted to a Nitch•Nitch liquid culture medium added with indole butyric acid such that the amount becomes $10^{-5}$M, and were passage cultured for two years. Into 100 ml volume Erlenmeyer flasks containing 20 ml of a Nitch•Nitch liquid culture medium added with phenyl alanine such that the phenyl alanine concentration becomes 0.050 mM, and with indole butyric acid such as its concentration becomes $10^{-5}$M, the adventitious roots thus obtained were transplanted, and shaking culture was started, Phenyl alanine was successively added to increase the phenyl alanine concentration every two days for 0.05 mM, and cultured for 3 weeks. After drying the adventitious roots obtained, they were extracted with 50 ml of basic chloroform-methanol liquid. Then, 40 ml of 1 N sulfuric acid were added thereto to transfer the alkaloid to the chloroform layer, which was concentrated under reduced pressure, and its alkaloid amount was analyzed on gas chromatography. The production amount of alkaloid in this case are shown in Table 7.

By the way, the analysis on gas chromatography was effected under the following conditions.
Column: Silicone OV-17 ( 1% ) on Chromosorb W ( Mesh 80 to 100 ) 3 mmØ × 1 m Glass column
Carrier gas: $N_2$
Column temperature: 200°C

Example 22

After treating the leaves of Duboisis myoporoides R. Br with 10% antiformine, they were put on a Linsmaier & Skoog (LS) agar culture medium containing $10^{-5}$M benzyl adenine. The stems of the Duboisia sapling, which were obtained from the sapling generated after one month and passage cultured for 40 days on the same culture medium, and not yet lignified, were cut to the length of about 1 to 2 cm, and after immersing in a suspension ($10^7$ pieces /ml) of Agrobacterium rhizogenes HRI-1, shake-cultured for 24 hours, were put on LS agar culture medium containing no plant hormone. After 3 weeks, adventitious roots

were generated from the cut part of the stem of the sapling. The roots were treated in the LS liquid culture medium containing 0.1 mg/ml of anpiciline for two days, and self proliferating Duboisia infected adventittious roots containing no bacteria were obtained. This Duboisia infected adventitious roots were passage cultured for one year in the LS liquid culture medium. This adventitious root (dried weight of 10 mg) was transplanted into an Erlenmeyer flask containing 20 ml of LS liquid culture medium added with L-phenyl alanine such that its concentration becomes 50 µM, and phenyl alanine was successively added such that the phenyl alanine concentration is increased every two days for 50 µM. After drying the adventitious root obtained by culturing for 3 weeks, it was treated in the same manner as in Example 1, and the result of analysis of the alkaloid is shown in Table 7.

Comparative Example 10

Except that a culture medium without phenyl alanine added was used in Example 21, the experiment was carried out in the same manner as in said Example, and the result is shown in Table 7.

Comparative Example 11

Except that phenyl alanine was added at the initial period of culture only to 2 mM in Example 21, the experiment was carried out in the same manner as in said Example, and the result is shown in Table 7.

Comparative Examples 12 and 13

Except that phenyl alanine was added in Example 21 at the initial period of culture only to 1.0 mM or 0.5 mM, the experiment was carried out in the same manner as in said Example, and the result is shown in Table 7.

When explanation is given on Example 21 and Comparative Example 13, although the total addition amount of phenyl alanine during the culture period in both experiments is the same as to be in either case as 0.50 mM, but in contrast to that phenyl alanine amount was divided in Example 21 for 10 times such that the addition amount is 0.050 mM in each time of culture, while in Comparative Example 13, all amount 0.5mM was at first given.

When the results of both cases are compared, it is known that, in Example 21, where the addition was carried out by dividing into plural times, although the growth amount of the adventitious root is almost similar to that in the case of Comparative Example 13, but the production amounts of scopolamine and hyoscyamine have increased.

Comparative Example 14

Except that the culture medium not added with phenyl alanine was used in Example 22, the experiment was carried out in the same manner as in said Example, and the result is shown in Table 7.

In the following, the alkaloid high productive cells and the method for obtaining them will be further concretely explained by referring to Examples.

Example 23

The leaves of Duboisia mypoloides R. Br cultivated in the herb garden of our company were cleaned, and immersed in a 10% ammoniacal water for 10 minutes, and after successively cleaned with sterilized water 3 times, they were cut to the length of about 1 cm and put on a Linsmaier & Skoog agar culture medium added with naphthalene acetic acid and benzyl adenine such as to become the concentration of $10^{-5}$M and $10^{-6}$M, and were cultured at 25°C for 30 days. The adventitious root generated at the same time with the formation of callus was cut out, and transplanted in a Nitch•Nitch liquid culture medium added with indole butyric acid such as to attain the concentration of $10^{-5}$M, and were passage cultured for 2 years.

The adventitious root (dried weight of 20 mg) thus obtained was transplanted and cultured in a plastic

laboratory dish having diameter of 9 cm and containing 20 ml of Nitch•Nitch agar culture medium added with parafluorophenyl alanine and indole butyric acid such as the concentration becomes, respectively, 0.3 mM and $10^{-5}$M. After one month, the growing root was transplanted on a Nitch•Nitch agar culture medium containing parafluorophenyl alanine and indole butyric acid respectively to the concentration of 0.3 mM and $10^{-5}$M, and further cultured on the same culture medium for one month, and an adventitious root which shows tolerance to parafluorophenyl alanine was obtained. This adventitious root (dried weight of 10 mg) was transplanted in a 100 ml volume Erlenmeyer flask containing 20 ml of Nitch•Nitch liquid culture medium added with indole butyric acid such as to make the concentration become $10^{-5}$M, and cultured for 3 weeks, and further, passage culture was repeated once more. The adventitious root obtained was dried, and the productivity of tropane alkaloids was measured.

The analysis of tropane alkaloids was carried out as follows. At first, dried adventitious root was extracted by use of 50 ml of basic chloroform-methanol liquid, and 40 ml of 1 N sulfuric acid was added thereto to replace the alkaloid layer to the sulfuric acid layer. Further, 2 ml of ammoniacal water and 40 ml of chloroform were added to replace the alkaloid into the chloroform layer, which was then concentrated under reduced pressure, and the alkaloid amount was measured on the gas chromatograph. The distribution of the alkaloid production amounts of the phenyl alanine analog tolerant stocks obtained at this time is shown in Fig. 1. By the way, the analysis on the gas chromatograph was effected under the following conditions.

Column: Silicone OV-17 (1%) on Chromosorb W (Mesh 80 to 100) 3 mmØ × 1 m Glass column
Carrier gas: $N_2$
Column temperature: 200° C

From these ones, the one having the production amount of tropane alkaloids of more than 70 mg/l was selected as the tropane alkaloid-high productive cell.

Comparative Example 15

Except that the culture medium not added with parafluorophenyl alanine was used in Example 23, the experiment was carried out in the same manner as in said Example 23, and the distribution of the alkaloid production amounts is shown in Fig. 2.

Fig. 1 is a diagram for showing the distribution of the alkaloid production amounts of the phenyl alanine analog tolerant stocks obtained in Example 1, and Fig. 2 is a diagram for showing the distribution of the alkaloid production amounts of the stocks obtained in Comparative Example 1.

As can be clearly seen in Figs. 1 and 2, the tropane alkaloid-high productive stocks are present in the parafluorophenyl alanine tolerant stocks at a high ratio.

In the following, explanation will be given more concretely on the method of producing tropane alkaloids by use of Datura arborea of the present invention by referring to Examples.

Example 24

The leaves of Datura arborea L. cultivated in the herb garden of our company was cleaned and immersed in a 10% antiformin liquid, and successively, after being cleaned with sterilized water for 3 times, they were cut into about 1 cm length, and were put on a Nitch•Nitch agar culture medium added with indole butyric acid such that its concentration becomes $10^{-5}$M, and were cultured at 25° C for 3 weeks.

Adventitious roots generated from the leave fractions were transplanted in a Nitch•Nitch liquid culture medium added with indole butyric acid such as its concentration becomes $10^{-5}$M, and were passage cultured for one year. The adventitious root (dried weight of 10 mg) was transplanted in a 100 ml Erlenmeyer flask containing 20 ml of Nitch•Nitch liquid culture medium added with indole butyric acid such that its concentration becomes $10^{-5}$M and was cultured for 3 weeks. After drying the adventitious root obtained, it was extracted with 50 ml of basic chloroform-methanol liquid, and then, 40 ml of 1 N sulfuric acid was added thereto to replace the alkaloid layer to the sulfuric acid layer. Further, 2 ml of ammoniacal water and 40 ml of chloroform were added to transfer alkaloid to the chloroform layer, and the resultant liquid was concentrated under reduced pressure, and the alkaloid amount was analyzed on the gas chromatograph. The production amount of alkaloid in this case is shown in Tanle 8.

By the way, the gas chlomatograph analysis was carried out under the following conditions.

Column: Silicone OV-17 (1%) on Chromosorb W (Mesh 80 to 100) 3 mmØ × 1 m Glass column
Carrier gas: $N_2$

Column temperature: 200° C

Example 25

The leaves of Datura arborea L. were treated with 10% antiformin, and after cleaning 3 times with stelirized water, they were immersed in a suspension ($10^{-7}$ pieces/ml) of Agrobacterium rhizogenes HRI-1 shake-cultured for 24 hours, and subsequently put on an LS agar culture medium without containing plant hormone. After 2 to 3 weeks, adventitious roots was generated from the leaves. These were treated in a Nitch•Nitch liquid culture medium containing 0.1 mg/ml of ampicilin for two days, and a self proliferating adventitious root containing no bacterium was obtained. This infected adventitious root dbtained from Datura was passage cultured for 6 months in a Nitch•Nitch liquid culture medium. This adventitious root (dried weight of 10 mg) was transplanted in a 100 ml volume Erlenmeyer flask containing 20 ml of Nitch•Nitch liquid culture medium without containing hormone, and was shake cultured for 3 weeks. The adventitious root obtained (dried weight of 120 mg) was dried and extracted by the same method as in Example 24, and the alkaloid amount was analyzed. The alkaloid productivity in this case is shown in Table 8.

Comparative Example 16

Except that Duboisia myoporoides was used as a plant in Example 24, the experiment was carried out in the same manner as in said Example, and the result is shown in Table 1.

Comparative Examples 17 to 19

Except that Scopolia japonica, Hyosyamus niger, and Atropa belladonna were used as plants, and a Nitch•Nitch liquid culture medium containing no hormone as culture medium in Example 24, the experiment was carried out in the same manner as in said Example, and the results are shown in Table 8.

Comparative Examples 20 to 22

Except that Datura innoxia, Datura stramonium, and Hyoscyamus niger were used as plants in Example 25, the experiment was carried out in the same manner as in said Example, and the results are shown in Table 8.

In the following, the method for separating and purifying tropane alkaloids of the present invention will be concretely explained by referring to Examples.

Example 26

After heating the culture mixture (as the dried root, 20g: scopolamine content 100 mg), obtained by the liquid tissue culture of the Duboisia adventitious root as it is, in a water bath of 60° C for 12 hours, the mixture was filtered to remove the adventitious root. To the alkaloid containing culture medium, was added 1 N NaOH to regulate the pH to 7.0. A counter current extracting equipment was used to send the alkaloid-containing culture medium at a rate of 500 ml/hr and isobutyl acetate at a rate of 250 ml/hr to the extracting equipment continuously, and when the mixture was stirred violently, the mixture was separated to an isobutyl acetate layer and a water layer. Anhydrous sodium sulfate was added to the isobutyl acetate layer containing extracted scopolamine to dehydrate it. When isobutyl acetate was distilled off under a reduced pressure of 20 to 30 mm Hg, 250 mg of syrup-like substance containing scopolamine, nicotine, and small amount of atropin was obtained. Then, after dissolving the syrup-like substance by adding 2g ethanol, 0.4 g of 47% HBr solution was added dropwise, and the mixture was shaken. After leaving the mixture to stand still at 5° C for one night, 62 mg of the objective scopolamine•3H2O•HBr was obtained. (Recovery percentage 62%. purity 99.0%).

Example 27

Except that the pH of the alkaloid-containing culture medium was made to 8.0 in Example 26, the experiment was carried out in the same manner as in Example 1, and 68 mg of scopolamine•$3H_2O$•HBr were obtained. (Recovery percentage 68%, purity 98.5%).

Example 28

Except that in place of isobutyl acetate to be used in counter current extraction, ethyl acetate was used, the experiment was carried out in the same manner as in Example 1, and 49 mg of scopolamine•$3H_2O$•HBr were obtained. (Recovery percentage 49%, purity 99.0%).

Example 29

Except that the callus (200g as a dried callus: scopolamine content 100 mg) obtained by the cell culture of scopolamine in place of the use of the adventitious root as the extraction raw material in Example 26, the experiment was carried out in the same manner as in Example 1, and 35 mg of scopolamine•$3H_2O$•HBr was obtained. (Recovery percentage 35%, purity 98.0%).

Comparative Example 23

Except that chloroform was used in place of isobutyl acetate as an extracting solvent in Example 26, the experiment was carried out in the same manner as in Example 1, and 66 mg of scopolamine 3H2O HBr was obtained. (Recovery percentage 59%, purity 95.5%).

Example 30

Except that the culture mixture (25g as a dried root: scopolamine content 100 mg) obtained by the liquid culture of Hyoscyamus adventitious root was used in place of the use of the adventitious root of Duboisia as the extraction raw material in Example 26, the experiment was carried out in the same manner as in Example 26, and 58 mg of scopolamine 3H2O HBr was obtained. (Recovery percentage 51%, purity 99.0%).

Example 31

Except that the culture mixture (15.4 g as a dried root: scopolamine content 100 mg) obtained by the liquid culture of Datura adventitious root was used in place of the use of Duboisia adventitious root as the extraction raw material in Example 26, the experiment was carried out in the same manner as in Example 26, and 51 mg of scopolamine 3H2O HBr was obtained. (Recovery percentage 51%, purity 98.8%).

Table 1

| Experiment | Additive | Addition amount (μM) | Growth amount (g/ℓ) | Scopolamine (mg/ℓ) | Hyoscyamine (mg/ℓ) |
|---|---|---|---|---|---|
| Comparative Example 1 | - | 0 | 5.7 | 50.9 | 22.1 |
| Example 1 | phnylalanyl metionine | 10 | 7.9 | 64.5 | 23.5 |
| " 2 | " | 100 | 4.9 | 82.1 | 41.0 |
| " 3 | " | 500 | 4.7 | 78.8 | 35.0 |

Table 2

| Experiment | Additive | Addition amount (μM) | Growth amount (g/ℓ) | Scopolamine (mg/ℓ) | Hyoscyamine (mg/ℓ) |
|---|---|---|---|---|---|
| Comparative Example 2 | - | 0 | 5.5 | 47.8 | 22.6 |
| Example 4 | diphenyl alanine | 1 | 4.3 | 59.6 | 18.7 |
| " 5 | " | 10 | 3.5 | 72.4 | 19.5 |
| " 6 | " | 100 | 2.4 | 48.4 | 16.8 |

Table 3

| Experiment | Additive | Addition amount (μM) | Growth amount (g/ℓ) | Scopolamine (mg/ℓ) | Hyoscyamine (mg/ℓ) |
|---|---|---|---|---|---|
| Comparative Example 3 | - | 0 | 5.8 | 46.3 | 19.4 |
| Example 7 | triphenyl alanine | 0.5 | 5.9 | 47.6 | 21.0 |
| " 8 | " | 1.0 | 5.4 | 54.8 | 23.5 |
| " 9 | " | 5.0 | 4.7 | 69.9 | 24.1 |
| " 10 | " | 10.0 | 3.2 | 65.4 | 22.6 |

Table 4

| Experiment | Additive | Addition amount (μM) | Addition period | Growth amount (g/ℓ) | Scopolamine (mg/ℓ) | Hyoscyamine (mg/ℓ) |
|---|---|---|---|---|---|---|
| Comparative Example 4 | - | 0 | - | 7.5 | 35.0 | 10.5 |
| " " 5 | 4-fluorophenyl alanine | 0.05 | Initial period of culture | 7.3 | 35.5 | 10.3 |
| Example 11 | " | 1.0 | " | 7.0 | 48.0 | 12.5 |
| " 12 | " | 3.0 | " | 6.0 | 60.5 | 13.8 |
| " 13 | " | 10.0 | " | 5.0 | 53.5 | 14.5 |
| " 14 | " | 30.0 | " | 4.3 | 52.0 | 13.8 |

Table 5

| Experiment | Additive | Addition amount (μM) | Addition period | Growth amount (g/ℓ) | Scopolamine (mg/ℓ) | Hyoscyamine (mg/ℓ) |
|---|---|---|---|---|---|---|
| Comparative Example 6 | - | 0 | - | 7.7 | 58.4 | 25.4 |
| " " 7 | 4-fluorophenyl alanine | 0.05 | Initial period of culture | 7.5 | 57.6 | 28.8 |
| Example 15 | " | 3.0 | " | 7.6 | 71.9 | 34.7 |
| " 16 | " | 10.0 | " | 7.2 | 85.1 | 36.3 |
| " 17 | " | 30.0 | " | 4.2 | 68.4 | 29.5 |

Table 6

| Experiment | Additive | Addition amount (μM) | Addition period | Growth amount (g/ℓ) | Scopolamine (mg/ℓ) | Hyoscyamine (mg/ℓ) |
|---|---|---|---|---|---|---|
| Comparative Example 8 | - | 0 | - | 7.5 | 52.3 | 28.1 |
| " " 9 | 4-fluorophenyl alanine | 0.5 | Initial period of culture | 7.6 | 54.2 | 25.3 |
| Example 18 | " | 3.0 | " | 7.3 | 64.7 | 29.9 |
| " 19 | " | 10.0 | " | 7.0 | 79.9 | 34.6 |
| " 20 | " | 30.0 | " | 5.2 | 55.6 | 26.7 |

Table 7

| Experiment | Additive | Addition amount (μM) | Addition period | Growth amount (g/ℓ) | Scopolamine (mg/ℓ) | Hyoscyamine (mg/ℓ) |
|---|---|---|---|---|---|---|
| Comparative Example 10 | - | 0 | - | 7.5 | 35.0 | 10.5 |
| " " 11 | Phenyl alanine | 2.0 | Initial period of culture | 3.5 | 13.5 | 5.5 |
| " " 12 | Phenyl alanine | 1.0 | " | 6.7 | 30.0 | 17.0 |
| " " 13 | Phenyl alanine | 0.50 | " | 7.1 | 32.5 | 10.0 |
| Example 21 | Phenyl alanine | 0.050 | Initial period of culture, 2nd day, 4th day, 6th day, 8th day, 10th day, 12th day, 14th day, 16th day, 18th day | 7.3 | 55.5 | 12.9 |
| " 22 | Phenyl alanine | 0.050 | " | 6.5 | 70.1 | 14.8 |
| Comparative Example 14 | - | 0 | - | 6.0 | 56.2 | 11.2 |

Table 8

| Experiment | Name of plant | Adventitious root deriving method | Growth amount (g/ℓ) | Scopolamine content (%) | Hyoscyamine content (%) | Content of nicotine and derivatives (%) |
|---|---|---|---|---|---|---|
| Example 24 | Datura arborea | Hormone | 6.0 | 0.85 | 0.58 | 0.0 |
| Comparative Example 16 | Duboisia myoporoides | " | 6.5 | 0.59 | 0.32 | 0.45 |
| " " 17 | Scopolia japonica | " | 3.2 | 0.05 | 0.16 | 0.0 |
| " " 18 | Hyoscyamus niger | " | 4.2 | 0.24 | 0.23 | 0.0 |
| " " 19 | Atropa belladonna | " | 4.5 | 0.15 | 0.24 | 0.0 |
| Example 25 | Datura arborea | Ag.rhizogenes | 6.5 | 1.41 | 0.75 | 0.0 |
| Comparative Example 20 | Datura innoxia | " | 4.5 | 0.02 | 0.85 | 0.0 |
| " " 21 | Datura stramonium | " | 4.5 | 0.05 | 0.56 | 0.0 |
| " " 22 | Hyoscyamus niger | " | 4.6 | 0.22 | 0.35 | 0.0 |

## Claims

1. A method for producing a tropane alkaloid which comprises the tissue culture of tissues or cells of a tropane alkaloid-producing plant in a medium containing an oligopeptide containing phenylalanine as a constituent amino acid or in a medium containing at least 1.0 μM of a phenylalanine analog.

2. A method for producing a tropane alkaloid which comprises the tissue culture of tissues or cells of a tropane alkaloid-producing plant in a medium containing phenylalanine and/or an analog thereof such that the amount of phenylalanine and/or analog added to the medium at any one time is below 0.5 mM and phenylalanine and/or an analog thereof is added a plurality of times over the culture period.

3. A method according to claim 1, wherein said oligopeptide is phenylalaninemethionine, diphenylalanine or triphenylalanine.

4. A method according to claim 1 or 2, wherein said phenylalanine analog is 4-fluorophenylalanine or 4-chlorophenylalanine.

5. A method according to any one of claims 1 to 4, wherein the tropane alkaloid-producing plant is **Duboisia myoporoides.**

6. A tropane alkaloid-hyperproducing cell derived from a tropane alkaloid-producing plant and showing tolerance to phenylalanine or an analog thereof.

7. A cell according to claim 6, wherein the tropane alkaloid-producing plant is **Duboisia myoporoides.**

8. A cell according to claim 6 or 7, wherein the phenylalanine analog is parafluorophenylalanine or thienylalanine.

9. A method for obtaining a tropane alkaloid-hyperproducing cell, which comprises preparing cells showing tolerance to phenylalanine or an analog thereof by culturing tissues or cells of a tropane alkaloid-producing plant in a medium containing phenylalanine or an analog thereof and selecting a tropane alkaloid-hyperproducing cell from the resulting tolerant cells.

10. A method according to claim 9, wherein the tropane alkaloid-producing plant is **Duboisia myoporoides.**

11. A method according to claim 9 or 10, wherein the phenylalanine analog is paraflurophenylalanine or thienylphenylalanine.

12. A method for producing a tropane alkaloid which comprises culturing an adventitious root of **Datura arborea L.**

13. A method for separating and purifying tropane alkaloid which comprises releasing a tropane alkaloid containing in a cell into a medium by heat-treating a tissue or cells of a tropane alkaloid-containing plant, adjusting the pH of said medium to 6.5 to 8.5, extracting a tropane alkaloid with aliphatic carboxylic acid ester from the pH-adjusted medium, removing the aliphatic carboxylic acid ester from the extract, treating the thus treated extract with a hydrogen halide to precipitate a tropane alkaloid as a salt of the hydrogen halide, and separating said salt.

FIG.1

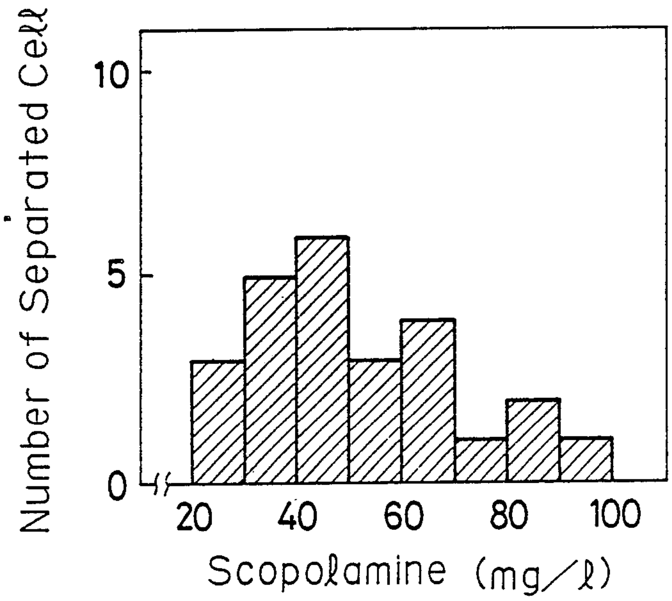
Number of Separated Cell
10
5
0
20
40
60
80
100
Scopolamine (mg/ℓ)

FIG.2

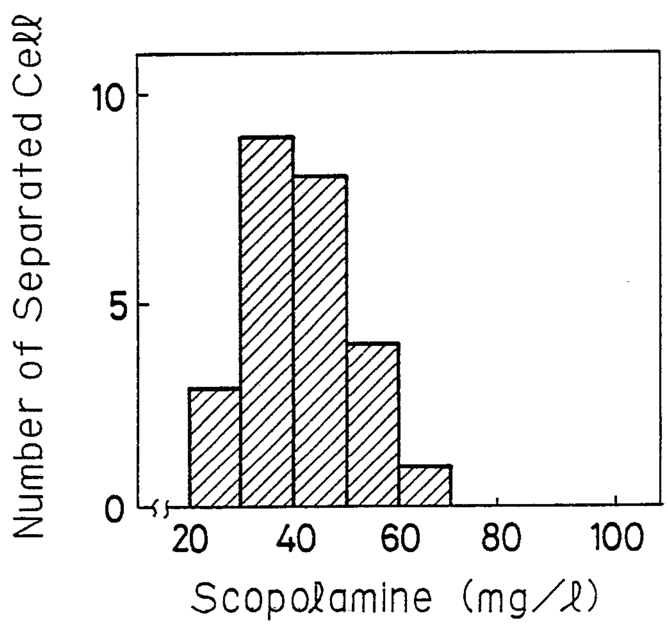
Number of Separated Cell
10
5
0
20
40
60
80
100
Scopolamine (mg/ℓ)